# FASCICULE DE BREVET EUROPEEN

(11) **EP 2 211 716 B1**
(45) Date de publication et mention de la délivrance du brevet: **10.04.2013**
(21) Numéro de dépôt: 08857368.8
(22) Date de dépôt: 26.09.2008
(51) Int. Cl.: A61B 5/151

(54) **DISPOSITIF DE PRELEVEMENT SANGUIN COMPORTANT AU MOINS UN FILTRE.**
BLUTPROBENAHMEVORRICHTUNG MIT MINDESTENS EINEM FILTER
BLOOD SAMPLING DEVICE COMPRISING AT LEAST ONE FILTER

(30) Priorité: 02.11.2007 FR 0707709
(43) Date de publication de la demande: 04.08.2010
(73) Titulaire: Commissariat à l'Énergie Atomique et aux Énergies Alternatives, 75015 Paris (FR)
(72) Inventeur: ROSTAING, Hervé, F-38420 Le Versoud (FR); PEPONNET, Christine, F-38170 Seyssinet (FR); POUTEAU, Patrick, F-38240 Meylan (FR); SOLLIER, Elodie, F-38000 Grenoble (FR)
(74) Mandataire: Reboussin, Yohann Mickaël Noël
(86) Numéro de dépôt international: PCT/FR2008/001341
(87) Numéro de publication internationale: WO 2009/071775

(56) Documents cités:
- EP-A- 0 336 483
- EP-A- 1 659 404
- JP-A- 2007 006 973
- US-A- 5 306 623

## Description

L'invention concerne un dispositif de prélèvement d'un échantillon sanguin en vue d'un diagnostic et incorporant au moins un filtre pour séparer une phase plasma du sang d'une phase cellulaire.

Il existe déjà des dispositifs de diagnostic "exportés" chez le patient, à l'extérieur des centres de soin. Ces dispositifs doivent être simples d'utilisation et l'échantillon auto-prélevé doit être mesuré sur place ou stabilisé pour être stocké plusieurs jours afin d'être envoyé (par la poste) à un centre de soins qui effectuera les mesures.

Cette procédure se développe car elle évite le déplacement des patients et permet de multiplier les tests souvent de manière non-invasive et très peu traumatisante. L'un des meilleurs exemples est le test du taux de glucose pour le diabète qui est totalement géré par le patient à partir de 1 µl de sang.

L'invention s'adresse plus particulièrement quoique non exclusivement à un dispositif de prélèvement en vue de connaître la composition en protéines d'un échantillon sanguin, notamment dans le cadre d'études épidémiologiques ou de recherche de biomarqueurs.

Pour cela, le sang doit être fractionné en une phase plasma contenant les protéines et une phase cellulaire. Dans cette application, les cellules et principalement les globules rouges doivent être exclus sans être lysés afin de ne pas perturber la mesure du spectre des protéines réalisée en utilisant un spectromètre de masse.

Le spectromètre n'étant pas intégrable dans le dispositif consommable, il faut, dans le cas où le patient n'est pas présent à proximité, stabiliser le plasma le temps du transport jusqu'au centre de mesure, en général plusieurs jours. Pour ce faire, le plasma est de préférence absorbé et séché sur un média spécifique.

D'une manière générale, la quasi-totalité des techniques de fragmentation que ce soit pour la transfusion ou l'analyse utilisent un principe de centrifugation du sang. En effet les cellules sanguines ont une densité légèrement supérieure à celle du plasma et peuvent donc être aisément et efficacement séparées avec des techniques de centrifugation classiques.

Cependant, cette technique nécessite un appareillage dédié et du personnel qualifié pour prélever l'échantillon de sang et le traiter. Les lieux de prélèvement et d'analyse sont souvent éloignés l'un de l'autre et les échantillons attendent souvent de nombreuses heures avant de pouvoir être analysés. Pendant ce temps de latence, les éléments constitutifs du sang (dont les protéines) se dégradent progressivement.

De plus, les techniques de prélèvement et de fragmentation actuelles sont conçues pour traiter plusieurs ml de sang alors que la plupart des tests ne nécessitent que quelques centaines de µl. Et l'extraction de plusieurs ml de sang est traumatisante pour une partie des patients et ne peut être répétée trop souvent.

Il existe un certain nombre de dispositifs connus qui sont aptes à séparer les cellules du sang pour des volumes de prélèvement se limitant à quelques centaines de microlitres de sang.

Dans ces dispositifs, la séparation est assurée par des filtres en matériaux fibreux, à savoir en fibre de verre, comme dans le Brevet US 5,364,533 (SANWA KAGAKU KEWKYUSHO) pour recueillir un volume de sang d'un millilitre environ ou bien comme dans la Demande de Brevet EP-633 808 (GOSTECHNOLOGY) dans laquelle les fibres de verre sont à l'état compressé.

Il est également connu d'utiliser un agent agglomérant ou agent agglutinant comme agent de séparation. Ceci est prévu à titre optionnel dans le Brevet US 5,364,533 précité et à titre principal dans les Brevets US 5,558,834 (BAYER Corp.) et US 6,106,732 (BINAX Services).

Ces techniques présentent les inconvénients suivants.

Tout d'abord, les agents agglutinants ont une sélectivité variable selon les types de protéines, ce qui créé un biais dans la représentativité de l'échantillon aussi sollicité.

D'autre part, les fibres, et plus particulièrement les fibres de verre, ne retiennent pas tous les globules, ce qui perturbe la mesure au spectromètre de masse pour lequel il est important d'avoir un plasma très clair, c'est-à-dire sans hémoglobine des globules rouges.

L'invention concerne ainsi un dispositif de prélèvement sanguin par capillarité selon la revendication 1.

Le dispositif de collection du sang peut être intégré au dispositif de diagnostic ou bien être séparé de celui-ci ;

La dimension des pores est avantageusement comprise entre 0,5 µ et 5 µ pour éviter la lyse des globules. La densité des pores peut être telle qu'ils occupent entre 10% et 90%, et plus particulièrement entre 40% et 80% et de préférence entre 50% et 80%, de la surface de la membrane.

Au moins une deuxième membrane de même dimension de pores et/ou une même densité de pores ou ayant une dimension de pores et/ou une densité de pore différente peut être superposée à la première. Le dispositif peut présenter, en amont de la première membrane dans le sens de circulation de sang à travers le filtre lors d'un prélèvement, un papier absorbant et/ou filtrant servant à repartir le sang sur la surface du filtre, ledit papier pouvant comporter des fibres notamment des fibres de verre assurant un filtrage partiel de la phase cellulaire ;

Selon une variante, il est particulièrement avantageux de disposer en aval de la ou des membranes du filtre, un papier absorbant apte après séchage à conserver les protéines.

Le papier absorbant peut présenter des traits de prédécoupe permettant de séparer des aliquots. Il a de préférence une section supérieure à celle du filtre, et peut alors présenter, en dehors du pourtour du filtre, des zones de préhension pouvant être touchées sans risque de contamination de l'échantillon prélevé.

Le pourtour du papier absorbant peut être structuré par marquage (encre ou colle imprégnée dans l'épaisseur) ou compressé pour faciliter une bonne répartition de la phase plasma recueillie.

Selon une autre variante, le dispositif présente au lieu de papier absorbant une région structurée en réseau, par exemple en un réseau de piliers, notamment en matière plastique ou en silicium, pour retenir la phase plasma par capillarité. Ce réseau peut également propager par capillarité le liquide filtré vers une zone d'analyse ou de stabilisation.

Le dispositif de collection du sang présente avantageusement au moins une aiguille dont la longueur L, par exemple comprise entre 200 µ et 2 mm, et de préférence entre 1 mm et 2 mm, est adaptée à un prélèvement dans le derme.

Le dispositif de collection peut comporter une matrice d'aiguilles de longueur L réparties sur la surface du filtre, ce qui permet de répartir sur le filtre le sang recueilli ;

Le dispositif peut comporter un anticoagulant sous forme lyophilisée, sèche ou humide qui revêt les parois du dispositif de collection en contact avec le sang et/ou au moins une paroi du filtre.

Le dispositif peut être logé dans un boîtier qui comporte, en aval du filtre, une enceinte sous vide. Le dispositif de collection peut comporter un film protecteur qui revêt la ou les aiguilles du dispositif de prélèvement, de manière dans ce cas à maintenir le vide.

Le dispositif peut comprendre un préfiltre dont le volume est dimensionné de sorte que le papier absorbant est toujours saturé en plasma.

Le dispositif, en forme de bandelette, peut comprendre un support de bandelette pour rigidifier l'ensemble du dispositif, par exemple de nature hydrophobe et non absorbant.

Le dispositif peut être agencé de sorte que l'extrémité du préfiltre dépasse du reste du dispositif, par exemple de l'ordre de 1mm.

L'invention sera mieux comprise à la lecture de la description ci-après, en liaison avec les dessins dans lesquels :
- la figure 1 représente un mode de réalisation de l'invention avec un dispositif de collection intégré présentant une matrice d'aiguilles ;
- la figure 2 représente le dispositif de la figure 1 sous boîtier, avec une enceinte à vide optionnelle ;
- la figure 3 illustre une variante de l'invention pour un collection avec une seule aiguille ;
- la figure 4 est une vue en coupe illustrant la zone du derme pertinente pour un prélèvement sans douleur ;
- la figure 5 illustre un exemple de membrane filtrante utilisable dans le cadre de l'invention ;
- la figure 6 représente une variante dans laquelle les aiguilles sont pleines ;
- la figure 7 illustre un mode de réalisation d'un papier absorbant utilisable selon l'invention ;
- les figures 8a à 8d illustrent un assemblage d'un dispositif selon l'invention ;
- les figures 9a et 9b représentent en coupe et en vue de dessus un autre mode de réalisation du dispositif selon l'invention ;
- les figures 10a à 10c illustrent un moyen séparé de collection par capillarité qui peut être utilisé avec le dispositif de prélèvement des figures 9a et 9b, la figure 11 illustrant le cas d'un moyen de collection intégré au dispositif des figures 9a et 9b ; et
- les figures 12a et 12b montrent une variante présentant un réseau de piliers agencés pour permettre un recueil de la phase plasma par capillarité ;
- les figures 13a à 13c montrent une autre variante de réalisation selon l'invention, respectivement en vues de coupe sur les figures 13a et 13b, et en vue de dessus sur la figure 13c.

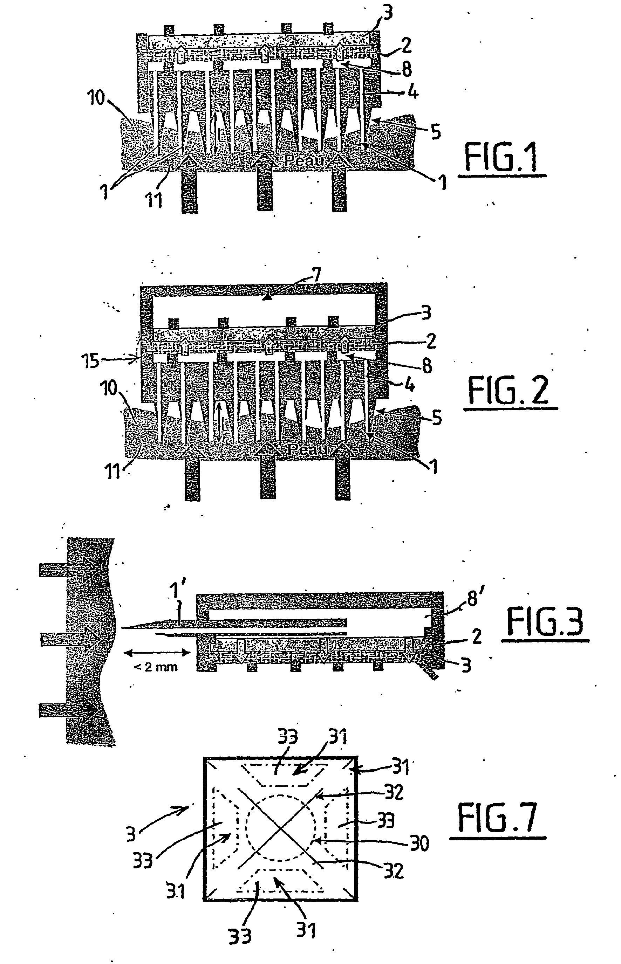

Le dispositif décrit ci-après permet en relation avec les figures 1 et 2 la séparation du sang total en une fraction de cellules et une fraction plasma. Plus précisément les cellules du sang sont retenues sur un filtre 2 par ségrégation dimensionnelle et sans l'utilisation dans le dispositif d'agent agglomérant notamment de type lectine ou agglutinine, le plasma passe à travers le filtre, puis il est stocké dans le moyen absorbant 3.

Il est à structure majoritairement 'verticale' (empilement en couches) et comprend une partie de collection du sang (qui est ou non intégré au dispositif), éventuellement un moyen d'aspiration du sang (Figure 2), éventuellement un moyen de répartition du sang sur le filtre, un élément de filtration, un moyen d'absorption du plasma sans dénaturation des protéines ainsi qu'un moyen pour enlever et sécher le plasma absorbé. Ce moyen de stockage peut être détachable. Le moyen de répartition peut être intégré à la partie collection qui est par exemple une matrice d'aiguilles, ou à la partie filtration sous la forme d'un préfiltre.

Le plasma séché est analysé ultérieurement (jusqu'à plusieurs semaines plus tard). Par exemple, il est envoyé par courrier pour faire une mesure du taux de protéine par spectrométrie de masse.

Le dispositif décrit est composé tout d'abord d'une aiguille ou une matrice d'aiguilles 1 qui a pour fonction de percer l'épiderme pour récolter une goutte de sang (10-150 µl) dans le derme. L'aiguille 1 peut avoir une profondeur optimale pour limiter la sensation de piqûre lors du prélèvement. Typiquement, une longueur d'aiguille est comprise entre 200 µm et 2 mm et son diamètre est de préférence inférieur à 2 mm. Les aiguilles 1 peuvent être creuses afin de directement récolter le sang au coeur du derme.

Une matrice d'aiguilles 1 est avantageuse pour répartir uniformément le sang sur le filtre comme expliqué plus loin dans la description et réduire la sensation de douleur en raccourcissant la longueur L des aiguilles. En effet, chaque aiguille a moins de sang à prélever donc la profondeur à atteindre au niveau du derme est moindre.

L'intérieur 4 des aiguilles 1 et/ou la chambre 8 à la surface du filtre et/ou le préfiltre 6 qui répartit le sang peut être pré rempli d'un agent anticoagulant et/ou d'un agent anti-hémolyse lyophilisé.

La diffusion du sang vers l'intérieur du dispositif peut être assurée par un moyen qui est la pression sanguine du donneur, par les forces de capillarité engendrées par la microstructuration (porosité par exemple) des différents constituants du dispositif ou encore par la mise en dépression d'une partie du dispositif (cavité 7 de la figure 2). Il y a un avantage certain en terme de lyse et de propreté de l'échantillon à prélever le sang directement dans la peau (approx. 1 mm de profondeur) en évitant tout contact avec la surface.

Le système de prélèvement-filtration peut être inclus dans un système de préhension du doigt (comme une pince à linge) qui a pour objet de maintenir en place la ou les aiguilles et le système durant le prélèvement et de favoriser un prélèvement rapide en comprimant le doigt du patient.

Le sang est mis en contact avec une (ou plusieurs) membrane(s) filtrante(s) 2 constituant le filtre. La membrane principale peut être un filtre commercial de n'importe quel type mais préférentiellement avec des pores de 0,1 à 5 µm et préférentiellement une membrane trouée fine (d'épaisseur entre 1 et 100 µm) et ayant une densité de trous aussi élevée que possible. De plus, le filtre principal 2 n'est ni un filtre en fibres de verre ni un filtre en cellulose en raison des défauts dus à la présence d'un filtrage par fibres. Un filtre en fibres de verre, comme on le verra ci-après, peut par contre être utilisé en amont de la membrane en tant que préfiltre 6 pour répartir le sang sur la membrane et effectue une préfiltration des globules. La partie filtrante 2 peut être une superposition de filtres de pores et/ou de structures différentes, mais toujours sans agent agglomérant ou agglutinant.

Cette partie est de préférence comprimée ou liée contre le moyen qui absorbe le plasma afin de maximiser la force de capillarité qui aspire le plasma à travers la membrane. La densité des pores peut être maximisée afin d'obtenir une plus grande quantité de plasma filtrée avant la saturation du filtre.

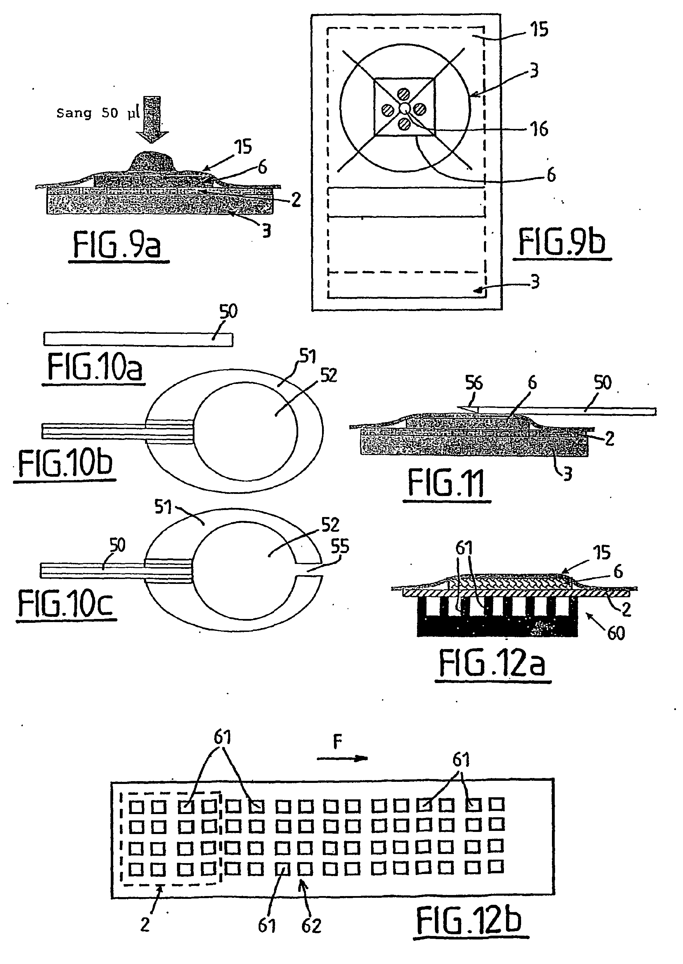

Le sang collecté est avantageusement réparti uniformément sur la surface du filtre. Cette fonction est assurée par :
- la répartition en matrice des aiguilles 1 qui assure une uniformité de l'approvisionnement du sang sur la surface, et/ou
- un préfiltre 6 de structure fibreuse qui se sature en sang par capillarité et répartit donc la goutte de sang sur toute la surface du filtre. Ce préfiltre peut ainsi aider à préfiltrer le sang avant une filtration beaucoup plus fine du filtre ; et/ou
- le remplissage d'un espace vide correspondant au volume de sang à traiter (10-150 µl et 50 µl de préférence) par capillarité sur les parois (figures 12a et 12b) ; et/ou
- l'aspiration par un vide (par exemple cavité 7, figure 2) qui permet de répartir le sang directement sur toute la membrane ; et/ou
- par étalement manuel à l'aide d'un capillaire étalonné.

Le plasma filtré est ensuite absorbé par un moyen de stockage à savoir un substrat hydrophile sur lequel le plasma s'étale, tel que le papier notamment "Whatman Protein Saver" 903 de la société Whatman ou bien encore une structure créant une force capillaire comme un réseau de piliers. Cette partie du dispositif a la propriété de pouvoir être séparée du reste du dispositif. Avantageusement, le moyen de stockage a un volume défini pour permettre une calibration précise du volume d'échantillon de plasma collecté.

Différentes techniques sont envisageables pour le séchage et la conservation de l'échantillon. Le séchage peut être à l'air libre, sous vide, par lyophilisation... La conservation peut être dans un sachet ou boîte individuelle avec un agent anti-humidité (type sachet dessicant), ou dans un conteneur pouvant également être sous-vide d'air toujours dans le but de favoriser la conservation des protéines.

Des tests ont été effectués avec un empilement (voir les figures 9a et 9b) composé d'un préfiltre 6 en fibres de verre (pores de 3µm, épaisseur entre 100 et 400µm), d'une membrane 2 présentant des trous de 1µm de diamètre (porosité entre 20% et 50%) et d'un papier 15 collant et compressant la partie filtrante 2 sur le moyen absorbant 3 (papier Protein Saver 903).

50 µl de sang humain total non dilué et anticoagulé par EDTA (hémotocrite : 45%) est disposé sur le dispositif. La membrane est maintenue manuellement au contact du papier absorbant pendant la filtration. Une quantité de plasma entre 7 et 11µl est filtrée en quelques minutes dans le papier absorbant 3.

Les figures 1 et 2 représentent une mise en oeuvre de l'invention, dans laquelle on remarque :
- L'intégration du moyen de collection (aiguille) au dispositif, avec des dimensions adaptées au volume de sang à prélever (50 µl).
- L'utilisation d'une membrane filtrante 2 par porosité sans agent agglomérant éventuellement couplée à un préfiltre pour filtrer le sang.
- Un moyen de calibrer la quantité de sang filtrée (préfiltre, chambre 8 avec un volume donné).
- L'utilisation d'un papier absorbant 3 non destructif pour les protéines pour récupérer le plasma par capillarité à travers le filtre 2. Un pré-découpage de ce papier (figure 7) permet de faciliter l'aliquotage et la structuration de celui-ci pour confiner le plasma dans une zone prédéfinie.

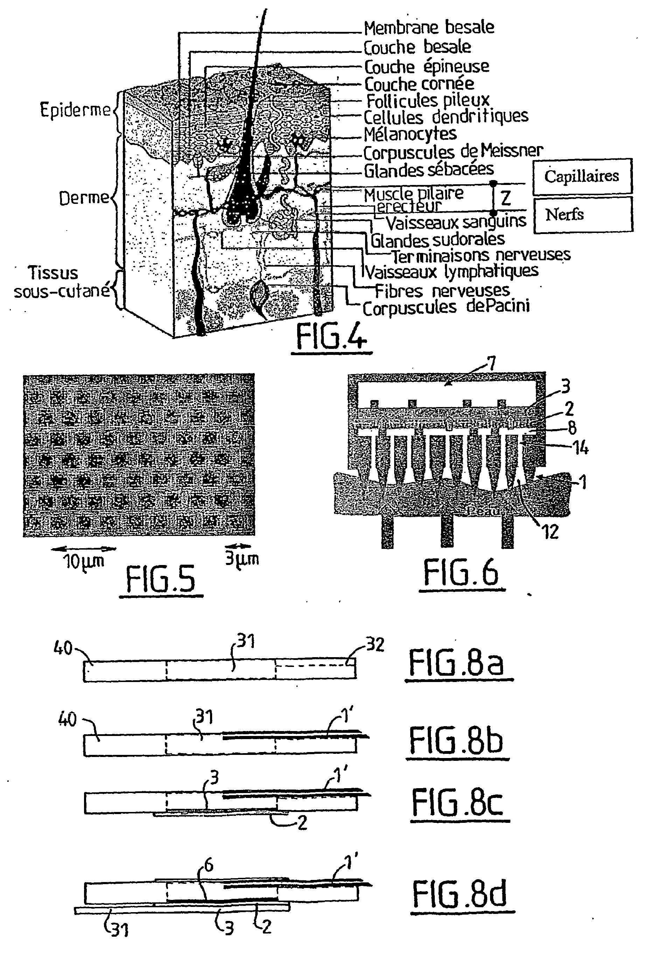

Dans l'exemple des figures 1 et 2, la surface du dispositif est composée d'une matrice d'aiguilles 1 (de quelques aiguilles à plus d'une centaine) ayant une longueur dépassante L telle que les aiguilles percent l'épiderme 10 mais ne rentrent pas profondément dans le derme 11 afin de pouvoir récupérer du sang en limitant fortement la sensation de douleur du patient (de 200 µm à 2 mm). L'illustration de la zone Z du derme (au niveau des capillaires et en amont des nerfs) qui est pertinente est donnée à la figure 4. De cette façon, la coagulation à la surface du doigt est très rapide et la marque plus discrète que pour un prélèvement standard.

Les aiguilles 1 peuvent être pleines ou trouées. Si elles sont trouées, le sang est alors directement collecté au coeur du derme ce qui présente un avantage certain au niveau de la qualité du sang. Si elles ne sont pas trouées, des trous 12 sont présents à la base des aiguilles comme illustré à la figure 6 pour canaliser le sang dans le dispositif à travers un canal 14 vers une cavité 8 jouxtant le filtre 2.

Grâce à la disposition des aiguilles 1 en matrice, la répartition du sang sur le filtre 2 se fait directement.

Les aiguilles 1 débouchent dans une cavité 8 de recueil.

La référence 2 désigne une membrane filtrante en amont de laquelle est éventuellement disposé un préfiltre 6.

La référence 3 désigne un media absorbant, éventuellement pelable.

Un anticoagulant sous forme lyophilisée, séchée, ou humide peut être présent sur les parois du canal 4 ou 14 des aiguilles et sur la surface amont du filtre 2 en contact avec le sang prélevé ;

Un film protecteur 5 des aiguilles 1 peut être prévu, lequel peut être enlevé avant utilisation, ou bien qui se rompt lorsque les aiguilles 1 pénètrent la peau.

La figure 2 représente un dispositif de prélèvement comme à la figure 1 mais qui est disposé dans un boîtier 15 comportant un volume sous vide 7.

Dans l'exemple de la figure 3, le dispositif est composé d'une seule aiguille 1' qui peut être un peu plus longue et large que dans le cas des matrices d'aiguilles car la totalité du sang doit être prélevée grâce à cette seule aiguille, et elle est en communication avec une cavité 8' bordant le filtre 2.

Le dispositif reste implanté dans la peau du patient durant tout le temps du remplissage de la chambre 7 ou 8. La fin du remplissage peut être déterminée grâce à un capot transparent ou avec un temps de prélèvement déterminé *a priori* (de quelques secondes à quelques minutes).

Une seconde possibilité est d'avoir un dispositif d'éjection et de retrait de l'aiguille intégré au dispositif afin de percer un trou pour récupérer le sang en évitant l'aspect traumatisant de garder une aiguille sous la peau.

Dans tous les cas, il est préférable d'utiliser un préfiltre 6 (par exemple un préfiltre en fibres de verre) en amont de la membrane principale 2 afin de répartir uniformément le sang sur la membrane principale 2. La couche constituant le préfiltre est suffisamment fine pour être quasi-saturée par la goutte de sang à prélever et assurer de cette façon une bonne répartition du sang sur le filtre 2.

Ainsi, on tirera avantage de la géométrie et de la structure du préfiltre 6 de façon à répartir le sang uniformément au contact de la membrane filtrante 2. Les globules contenus dans la sang sont de fait préférentiellement retenus dans le préfiltre 6, lequel limite, retient, lesdits globules, retardant ainsi leur mise au contact de la membrane ; ceci permet d'écarter tout risque de lyse prématurée des globules.

La membrane filtrante 2 est définie comme un film fin d'épaisseur comme entre 0,2µm et 100µm, le choix de l'épaisseur étant fonction de la non lyse des cellules filtrées. La membrane peut être par exemple en polycarbonate, en cellulose, en silicium, ou bien en oxyde de silicium. Elle comporte des trous de diamètre préférentiellement compris entre 0,1µm et 5µm, ayant leur axe préférentiellement perpendiculaire à la surface de la membrane 2. La porosité est ici définie comme étant une densité surfacique, à savoir le rapport entre la surface des trous ou pores et la surface totale de la membrane (et non de manière volumique comme c'est habituellement le cas). Elle est comprise entre 10% et 90% et de préférence entre 40% et 50%.

### Densité surfacique de pores du filtre :

Soit un filtre standard dont on estime la densité des pores (de 1 µm de diamètre) à 15 pour 100 µm², soit environ 12 % de la surface du filtre. Le diamètre du filtre est par exemple de 25 mm, ce qui représente 74 millions de trous de 1 µm de diamètre dans le filtre.

Le sang est composé de (données communément admises) :
- globules rouges : 5.10⁹ /ml
- Plaquettes : 3.10⁸ /ml
- globules blancs: 3.10⁶ /mL

Soit pour une goutte de 50 µl :
- globules rouges : 2.5.10⁸
- Plaquettes : 1.5.10⁷
- globules blancs : 0,35.10⁶,
   soit un total de 2,65.10⁸ cellules à filtrer c'est-à-dire 265 millions de cellules.
- Il y a dans cet exemple 3,6 fois plus de cellules à filtrer que de pores sur le filtre. Donc le filtre se bouche avant que tout le plasma ne le traverse, sauf à superposer plusieurs filtres.
- Avec un même filtre monocouche ayant une densité de pore supérieure ou égale à 43 %, soit 3,6 fois plus, la totalité du plasma peut être filtrée.

La figure 5 est un exemple de filtre à membrane silicium avec une grande densité de pores (>70%) dont le diamètre est de 1 µ,.

Pour l'application envisagée, le moyen de stockage est un substrat de préférence hydrophile sur lequel le plasma s'étale (par exemple type "Protein Saver 903 de Whatman") et a avantageusement la propriété de conserver les protéines après séchage.

L'épaisseur du moyen de stockage est maîtrisée en fonction de la quantité de plasma prélevé, de façon à permettre une répartition homogène du plasma filtré sur le papier absorbant.

Le papier absorbant peut être troué au centre pour permettre à une aiguille de trouer directement la peau sans détériorer les filtres et le papier absorbant.

Le moyen de recueil du plasma peut aussi être une surface non absorbante mais structurée pour recevoir 10 µl de plasma, par exemple un réseau de piliers plastiques ou silicium ayant un volume d'environ 10 µl et ayant de bonnes propriétés d'aspiration par capillarité (de préférence hydrophile).

La figure 7 illustre un papier absorbant qui convient particulièrement. Sa section est plus grande que le contour 20 du filtre 2, ce qui permet de disposer de zones 31 pouvant être touchées sans risque de contamination et qui permettent un marquage des échantillons par exemple par code barre. Il présente des traits de pré-découpe 32 permettant de séparer plusieurs échantillons (aliquotage). Pour assurer un aliquotage simple du plasma extrait, le papier est prédécoupé avantageusement par les traits 32 pour pouvoir séparer le plasma extrait en plusieurs aliquots de quantité comparable. Le pré-découpage des papiers est telle que l'aliquotage se fait simplement sans contact avec la partie où le plasma est absorbé et sans utilisation d'un outil quelconque. Le papier absorbant peut également avoir une épaisseur assez faible qui favorise un étalement homogène du plasma sur celui-ci et une surface structurée (compression locale du papier) afin de confiner et d'orienter l'étalement du plasma. La partie de préhension du papier (en dehors du contour 30 du filtre 2) peut de plus contenir une zone d'identification de l'échantillon (un code barre par exemple, une zone d'écriture, une bandelette RFID...) au recto ou au verso de l'échantillon.

Un mode de réalisation est décrit ci-après en relation avec la figure 8.

Le point de départ est dans ce cas une plaque plastique 40 de 1-2 mm d'épaisseur comprenant un trou 31 au centre dont le diamètre est inférieur à celui du filtre 2, et une rainure 32 partant du centre vers le bord du dispositif. Une aiguille 1' est tout d'abord collée dans la rainure 32. Le filtre principal 2 est collé sous la plaque plastique et le papier filtrant ou préfiltre 6 servant à répartir le sang sur la surface du filtre 2 est posé sur le filtre principal 2. Puis, le papier 3 absorbant le plasma est collé contre le filtre principal 2 sous le dispositif. Un film protecteur peut être également collé au dessus du dispositif ainsi réalisé.

Le dispositif représenté aux figures 9a et 9b présente une membrane filtrante 2 ayant une porosité de 1 µm qui est recouverte d'un préfiltre 6 en fibres de verre de porosité sensiblement égale à 3µm qui aspire et répartit le sang sur la membrane 2 tout en préfiltrant une partie des cellules. L'ensemble, qui comporte également un papier absorbant 3, est compressé par un adhésif 15 qui présente, au dessus du préfiltre 6, une ouverture 16 de passage du sang.

Une fois la filtration effectuée, l'adhésif est pelé.

La membrane 2 et le préfiltre 6 qui sont collés à l'adhésif sont donc enlevés en même temps que ce dernier à la fin du prélèvement, pour libérer le papier absorbant 3.

La figure 10a représente un capillaire 50 de collection de sang permettant de collecter un volume déterminé de sang après avoir piqué le derme d'un patient et qui est couplé avec une poire souple 51 présentant une cavité 52 (figure 10b) pour pipeter et vider la goutte de sang. La figure 10c montre une variante dans laquelle la poire 51 est trouée en 55 à son extrémité opposée au capillaire 50 pour aspirer le sang par capillarité et le vider en pressant sur la poire 51. Le capillaire 50 d'un volume de 50 à 100µl, éventuellement traité par un anticoagulant (de préférence l'EDTA) est utilisé comme intermédiaire. Il permet de récolter un volume précis de sang en se remplissant, après piqûre, par capillarité (donc sans bulle) et de vider le contenu directement sur le dispositif filtrant, soit à l'aide de la poire 51 ou comme décrit ci-après en relation avec la figure 11.

La figure 11 montre un capillaire 50 se terminant par une lancette 56 qui est intégré au dispositif des figures 9a et 9b. Le remplissage du capillaire 50 se fait par capillarité, et lorsqu'il est plein, le sang rentre en contact avec le préfiltre 6, et le sang est aspiré par capillarité par le préfiltre 6 et le capillaire 50 se vide progressivement à travers la lancette 56.

Le dispositif représenté aux figures 12a et 12b est semblable au dispositif des figures 9a et 9b sauf en ce que le recueil du sang est réalisé par un réseau 60 de piliers 61 en silicium de section 50µm x 50µm séparés entre eux par un intervalle de 50µm. Les piliers ont ici une profondeur de 200µm.

La figure 12b montre la zone de filtration 62 et le réseau 60 de piliers 61 pour le recueil de plasma, lequel se propage par capillarité dans le sens de la flèche F. Si les piliers 61 sont situés seulement en face du filtre 2, alors le plasma est retenu par capillarité sans le déplacement ci-dessus.

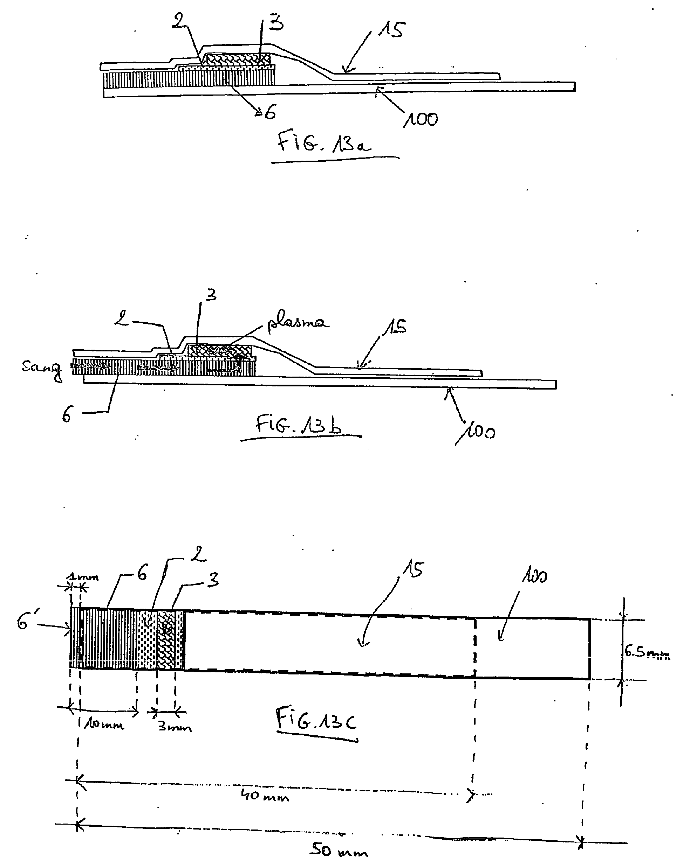

Le mode de réalisation représenté sur les figures 13a à 13c présente un dispositif en forme de bandelette comportant, un support de bandelette 100, un préfiltre 6 monté sur le support de bandelette 100, une membrane 2 montée sur le préfiltre 6, un papier absorbant 3 monté sur la membrane 2, ainsi qu'un adhésif pelable 15 en contact avec le papier absorbant.

Le préfiltre 6 vise à collecter le sang au niveau au niveau de la ponction capillaire, par son extrémité 6'. L'extrémité 6' du préfiltre dépasse légèrement du reste de la bandelette, par exemple de l'ordre de 1 mm. Le volume propre du préfiltre 6 lui permet de collecter une quantité de sang suffisante permettant une filtration des cellules de sang, et un remplissage optimal du papier absorbant 3 en plasma. On peut par exemple envisager que le préfiltre 6 soit un Fusion 5 de Whatman.

La membrane filtrante 2, formant barrière aux cellules de sang n'ayant pu être préfiltrées, est par exemple une membrane GE dont les pores ont un diamètre de 1 µm (le diamètre des pores est toujours choisi de sorte le plasma puisse circuler facilement et que la force capillaire du papier absorbant 3 ne lyse pas les cellules contre les pores de la membrane).

Le papier absorbant 3 présente des dimensions choisies pour se saturer avec un certain volume de plasma, typiquement de l'ordre de 5 à 6 µl. Il peut par exemple être réalisé par un Whatman Protein Saver 903.

Le papier absorbant 3 est solidaire de l'adhésif 15, qui est pelé après la filtration, de manière analogue à certains modes de réalisation de l'invention. L'autre face de l'adhésif 15 peut comprendre une surface imprimable, par exemple pour y mettre un code barre afin d'assurer la traçabilité du prélèvement de sang effectué. On peut également prévoir que cette autre face de l'adhésif soit faite ou comprenne un revêtement qui se colore au contact du plasma, de sorte que l'utilisateur puisse s'assurer du bon fonctionnement du dispositif.

Le support de bandelette 100 permet de rigidifier l'ensemble du dispositif, et peut par exemple être réalisé en papier cartonné, de type Bristol par exemple ; et avantageusement en matériau non absorbant de nature hydrophobe, par opposition aux filtres et préfiltre.

Le préfiltre 6 est dimensionné pour que la saturation du papier absorbant 3 en plasma ait toujours lieu afin de garantir un volume constant de plasma collecté, et ce quel que soit le patient (le taux d'hématocrite ou la viscosité du sang peuvent varier d'un patient à l'autre).

On comprend également que les matériaux sont choisis pour que l'adhésif 15 puisse être pelé sans arracher les différentes couches de l'empilement (support, préfiltre, membrane, papier absorbant).

Le dispositif peut présenter une largeur de 5 à 7mm : cette largeur correspond donc à celle de tous les éléments du dispositif. En revanche, la longueur de tous les éléments varie en fonction des besoins (volume de sang à prélever, volume de plasma à aliquoter).

Par exemple, le dispositif illustré sur ces figures permet de prélever 40µl de sang (avec un préfiltre de largeur 6.5mm et d'une longueur de 10mm), et de collecter 5.6µl de plasma (avec un papier absorbant 3 de largeur 6.5mm, et de longueur 3mm). Ces dimensions sont reportées sur la figure 13c, avec les dimensions afférentes de l'adhésif 15 (à savoir 40mm) et du support 100 (à savoir 50mm).

Le fonctionnement du dispositif est plus précisément illustré sur la figure 13b.

Le dispositif présenté tire avantage d'une rétention préalable opérée dans le préfiltre : le sang parvenant par la tranche 6', la mise au contact du sang avec la membrane 2 n'intervient qu'après un temps de migration par capillarité, la membrane 2 étant dimensionnée pour ne couvrir que partiellement la surface du préfiltre 6. Ainsi, il s'opère dans le préfiltre 6 une mobilité préférentielle de ses constituants (les protéines sont plus mobiles que les globules), favorisant la filtration et la ségrégation des constituants du sang.

En d'autres termes, la migration du sang par capillarité au sein du préfiltre 6 se fait essentiellement sur la longueur de la bandelette, ce temps de migration est mis à profit pour filtrer en avance de phase l'arrivée des constituants du sang sur la membrane 2, prévenant un risque de saturation de ladite membrane.

Le patient se pique tout d'abord un doigt avec une lancette, puis applique l'extrémité 6' du préfiltre 6, qui dépasse légèrement du reste du dispositif. Le préfiltre 6 se remplit de sang par capillarité. Une fois le préfiltre 6 rempli de sang, on observe le mouillage du papier absorbant 3 qui s'effectue également par capillarité. Le dispositif illustré sur les figures 13a à 13c est ainsi disposé que le passage du sang s'effectue de bas en haut (par référence à la gravité), et par capillarité.

Une fois le prélèvement terminé, il faut attendre environ deux minutes avant de peler la partie adhésive.

## Revendications

1. Dispositif de prélèvement sanguin par capillarité pour séparer une phase plasma du sang d'une phase cellulaire, le dispositif incorporant un filtre, et en aval de celui-ci, un matériau absorbant (3), le filtre comportant une première membrane (2) ayant des pores de dimension comprise entre 0,1 µm et 5 µm, cette membrane étant dépourvue d'agent agglomérant,
**caractérisé en ce que** le dispositif présente une forme de bandelette et le filtre comporte successivement,
- un préfiltre (6) pour répartir le sang sur la surface de la membrane (2), ce préfiltre (6) étant apte à collecter du sang au niveau de la ponction capillaire par son extrémité (6'), et
- ladite membrane (2), cette dernière ne couvrant que partiellement la surface du préfiltre (6),
de sorte que la migration du sang par capillarité au sein du préfiltre (6) s'effectue essentiellement sur la longueur de la bandelette avant que le sang ne soit mis au contact de ladite membrane (2).

2. Dispositif selon la revendication 1, **caractérisé en ce que** la dimension des pores est comprise entre 0,5 µm et 5 µm.

3. Dispositif selon une des revendications 1 ou 2, **caractérisé en ce que** la densité des pores est telle qu'ils occupent entre 10% et 90% de la surface de la membrane (2) du filtre.

4. Dispositif selon la revendication 3, **caractérisé en ce que** la densité des pores est telle qu'ils occupent entre 40% et 80% de la surface de la membrane, et de préférence entre 50% et 80% de la surface de la membrane.

5. Dispositif selon une des revendications précédentes, **caractérisé en ce que** le papier absorbant et/ou filtrant dit préfiltre (6) comporte des fibres notamment des fibres de verre assurant un filtrage partiel.

6. Dispositif selon une des revendications précédentes, **caractérisé en ce que** le matériau absorbant (3) est solidaire d'un adhésif (15) pelable.

7. Dispositif selon une des revendications précédentes, **caractérisé en ce que** le volume du préfiltre (6) est dimensionné de sorte que le papier absorbant (3) est toujours saturé en plasma.

8. Dispositif selon une des revendications précédentes, **caractérisé en ce qu'**il comprend un support (100) de bandelette pour rigidifier l'ensemble du dispositif, par exemple de nature hydrophobe et non absorbant.

9. Dispositif selon une des revendications précédentes, **caractérisé en ce que** l'extrémité (6') dépasse légèrement du reste de la bandelette (100), par exemple de l'ordre de 1 mm.

## Claims

1. Capillary-action blood sampling device for separating a blood plasma phase from a cellular phase, the device incorporating a filter and, downstream thereof, an absorbent material (3), the filter comprising a first membrane (2) having pores between 0.1 µm and 5 µm in size, this membrane being free from agglomerating agent,
**characterised in that** the device is in the form of a strip and the filter comprises, successively,
- a pre-filter (6) for distributing the blood over the surface of the membrane (2), this pre-filter (2) being adapted to collect blood at the capillary puncture site through its end (6'), and
- said membrane (2), the latter only partially covering the surface of the pre-filter (6),
so that the migration of the blood by capillarity within the pre-filter (6) takes place essentially over the length of the strip before the blood is brought into contact with the said membrane (2).

2. Device according to claim 1, **characterised in that** the dimension of the pores is between 0.5 µm and 5 µm.

3. Device according to one of claims 1 or 2, **characterised in that** the density of the pores is such that they take up between 10% and 90% of the surface of the membrane (2) of the filter.

4. Device according to claim 3, **characterised in that** the density of the pores is such that they take up between 40% and 80% of the surface of the membrane, and preferably between 50% and 80% of the surface of the membrane.

5. Device according to one of the preceding claims, **characterised in that** the absorbent and/or filtering paper known as the pre-filter (6) comprises fibres, notably glass fibres, which provide partial filtering.

6. Device according to one of the preceding claims, **characterised in that** the absorbent material (3) is attached to a peelable adhesive (15).

7. Device according to one of the preceding claims, **characterised in that** the volume of the pre-filter (6) is of such a size that the absorbent paper (3) is always saturated with plasma.

8. Device according to one of the preceding claims, **characterised in that** it comprises a support (100) for the strip for strengthening the device as a whole, for example of a water-repellent and non-absorbent nature.

9. Device according to one of the preceding claims, **characterised in that** the end (6') projects slightly past the remainder of the strip (100), for example by the order of 1 mm.

## Patentansprüche

1. Vorrichtung zur Blutentnahme durch Kapillarität zum Trennen einer Plasmaphase des Bluts von einer zellulären Phase, wobei die Vorrichtung ein Filter und stromab davon ein Absorbtionsmaterial (3) enthält, wobei das Filter eine erste Membran (2) mit Poren in einer Größenordnung von 0,1 µm und 5 µm umfasst, wobei die Membran frei von Agglomerationsmittel ist, **dadurch gekennzeichnet, dass** die Vorrichtung Streifenform aufweist und dass das Filter aufeinanderfolgend umfasst:
- ein Vorfilter (6), um das Blut auf der Oberfläche der Membran (2) zu verteilen, wobei das Vorfilter (6) geeignet ist, das Blut an der kapillaren Punktion mit seinem Ende (6') aufzunehmen, und
- die Membran (2), wobei letztere die Oberfläche des Vorfilters (6) nur teilweise bedeckt,
so dass die Migration des Bluts durch Kapillarität innerhalb des Vorfilters (6) im Wesentlichen in Längsrichtung des Streifens erfolgt, bevor das Blut mit der Membran (2) in Kontakt gebracht wird.

2. Vorrichtung nach Anspruch 1, **dadurch gekennzeichnet, dass** die Größenordnung der Poren zwischen 0,5 µm und 5 µm liegt.

3. Vorrichtung nach einem der Ansprüche 1 oder 2, **dadurch gekennzeichnet, dass** die Dichte der Poren derart ist, dass sie zwischen 10% und 90% der Oberfläche der Membran (2) des Filters einnehmen.

4. Vorrichtung nach Anspruch 3, **dadurch gekennzeichnet, dass** die Dichte der Poren derart ist, dass sie zwischen 40% und 80% der Oberfläche der Membran und vorzugsweise zwischen 50% und 80% der Oberfläche der Membran einnehmen.

5. Vorrichtung nach einem der vorhergehenden Ansprüche, **dadurch gekennzeichnet, dass** das als Vorfilter bezeichnete saugfähige Papier und/oder Filterpapier Fasern, insbesondere Glasfasern beinhaltet, die eine partielle Filtration sicherstellen.

6. Vorrichtung nach einem der vorhergehenden Ansprüche, **dadurch gekennzeichnet, dass** das Absorptionsmaterial (3) mit einem abziehbaren Klebemittel (15) verbunden ist.

7. Vorrichtung nach einem der vorhergehenden Ansprüche, **dadurch gekennzeichnet, dass** das Volumen des Vorfilters (6) so dimensioniert ist, dass das saugfähige Papier (3) immer in Plasma gesättigt ist.

8. Vorrichtung nach einem der vorhergehenden Ansprüche, **dadurch gekennzeichnet, dass** sie einen Streifenträger (100) umfasst, um die Vorrichtung als Ganzes zu versteifen, beispielsweise hydrophober und nichtabsorbierender Art.

9. Vorrichtung nach einem der vorhergehenden Ansprüche, **dadurch gekennzeichnet, dass** das Ende (6') leicht über den Rest des Streifens (100) hervorsteht, beispielsweise um etwa 1 mm.
